# EUROPEAN PATENT SPECIFICATION

(11) **EP 2 398 564 B1**
(45) Date of publication and mention of the grant of the patent: **10.06.2015**
(21) Application number: 10743464.9
(22) Date of filing: 11.02.2010
(51) Int. Cl.: A63B 24/00, A61N 5/06, A61N 1/32, A61B 18/00

(54) **A METHOD FOR BODY TONING AND AN INTEGRATED DATA MANAGEMENT SYSTEM FOR THE SAME**
KÖRPERSTRAFFUNGSVERFAHREN UND INTEGRIERTES DATENVERWALTUNGSSYSTEM DAFÜR
PROCÉDÉ DE TONIFICATION CORPORELLE ET SON SYSTÈME INTÉGRÉ DE GESTION DE DONNÉES

(30) Priority: 18.02.2009 US 388501
(43) Date of publication of application: 28.12.2011
(73) Proprietor: Syneron Medical Ltd., 20692 Yoqneam Illit (IL)
(72) Inventor: ECKHOUSE, Shimon, 34980 Haifa (IL); FLYASH, Lion, 17512 Nazareth Illit (IL)
(74) Representative: O'Neill, Brian
(86) International application number: PCT/IL2010/000121
(87) International publication number: WO 2010/095126

(56) References cited:
- WO-A1-2006/012752
- US-A- 4 602 280
- US-A1- 2003 032 950
- US-A1- 2005 177 139
- US-A1- 2007 027 411
- US-A1- 2007 027 411
- US-A1- 2008 004 678
- US-B2- 7 090 649

## Description

### TECHNICAL FIELD

The method disclosed herein and the system of the invention relate to the field of body toning and in particular to the field of management and operation of body toning devices.

### BACKGROUND

External appearance is of concern practically to every person. In recent years, methods and apparatuses have been developed for different skin toning and cosmetic treatments. Among these methods and apparatuses are hair removal, treatment of vascular lesions, wrinkle removal, body shaping, fat removal or reduction and skin rejuvenation. In these treatments a volume of skin or tissue to be treated is heated to a temperature that is sufficiently high as to achieve a desired effect. Such temperature is typically in the range of 38-60 degrees Celsius.

One method that has been used for heating the skin is application to the skin of pulsed or continuous radio-frequency (RF) energy. In this method, electrodes are applied to the skin and an RF voltage in continuous or pulse mode is applied across the electrodes. The properties of the RF voltage are selected so as to generate an RF current in the tissue to be treated, current which heats the tissue to the required temperature.

Concurrently, a number of light based skin surface or deeper skin layer treatments have been developed. These treatments usually employ a laser, a LED, a Xenon lamp (Intense Pulsed Light or IPL) or incandescent lamp radiation to expose a surface of skin where vascular lesions, varicose veins, acne, mole marks and similar disorders are present. The optical radiation may be of a single wavelength or include several wavelengths. The wavelengths are selected to be optimal for the color of the contrasted component of the target, and are typically in the range of 400 to 1800 nm.

Reduction of subcutaneous fat layers, or adipose tissue, is another skin treatment for which there is a growing demand. Among the different physical therapies available, the application of ultrasound is emerging as another adipose tissue removal and body shaping technology. Methods associated with this technology are based on the delivery of a dose of electromagnetic energy (RF) or ultrasound waves through the skin of a recipient into the subcutaneous adipose tissue to a volume of tissue to be treated.

WO 2006/012752 discloses a therapy device, including a body, an energy source disposed on the body for emitting a desired wavelength of electromagnetic radiation, and a material dispensing system disposed on the body for dispensing a desired material for use with the device. Also disclosed are accessories and compositions used with the therapy device, including interchangeable energy source-containing heads and interchangeable material containers. Methods for using the therapy device are also disclosed. US 2008/004678 discloses a method and system for heating a skin area surface of an individual from an initial temperature to a treatment temperature in a treatment time period exceeding 0.5 sec, where the treatment temperature is in the range of 40°C - 60°C. An RF generator provides a continuous wave RF voltage energy or a quasi-continuous wave RF voltage across first and second electrodes, where at least the first electrode is associated with an applicator that is displaced over the skin surface. The system further includes a skin temperature measuring device or an applicator displacement speed measuring device; and a CPU that monitors a skin temperature or an applicator displacement speed. The CPU turns off the RF energy when the skin temperature is above a predetermined temperature or the displacement speed of the applicator is below a predetermined speed, in order to prevent overheating of the skin.

The above described equipment is both costly and bulky, and it is typically operated in an ambulatory set-up by a qualified operator and frequently requires presence of medical personnel specialized in such treatments or toning. Recently, equipment allowing application of any one of the listed above treatments or a combination of them by a non-professional person in a conventional residential setting has been developed. When such equipment is in use, the user has no means for tracking the toning progress, select most appropriate for him/her tissue affecting energy parameters, apply the energy in the most effective way to the segments of tissue to be treated or toned.

There is a need for an integrated system that would collect the toning process parameters related to the application of tissue affecting energy to the user skin or tissue, analyze it, and recommend to the user current toning session tissue affecting energy parameters. There is also a need in a device capable of collecting such data and communicating it to the system as well as receiving and operating according to the recommended tissue affecting energy parameters.

### BRIEF SUMMARY

According to the invention, there is provided a personal tissue toning system as specified in claim 1. The problem of tracking the toning history of a person (subject) and determining parameters of the current toning session can be solved by recording and storing into the memory of a toning device, or a computer or some other processing device, parameters of each of the preceding tissue toning procedures, as well as segments of the subject body affected by such toning procedures. Next, the recorded data can be analyzed and one or more protocols related to the current tissue toning session can then be derived. Concurrently, with the protocol, recommendations on skin care options, new products adapted for skin care and complementary to the skin/tissue toning, and any other skin care news that may be relevant to the subject skin care can be identified and provided.

### GLOSSARY

The terms "tissue" or "skin" as used in the present disclosure have the same meaning and are used interchangeable through the text of the disclosure.

The term "tissue affecting energy" as used in the present disclosure means energy capable of causing a change in the tissue, or enabling such change. Such energy for example, may be RF energy, optical radiation in visible or invisible part of electromagnetic spectrum, ultrasound waves energy, and kinetic energy provided by a massaging device.

The term "tissue toning device" as used in the present disclosure means any device providing energy affecting the tissue. Such device for example, may apply to the tissue RF energy, optical radiation existing in the visible or the invisible part of spectrum, ultrasound waves energy, kinetic energy provided by a massaging device or some other source of energy.

The term "tissue toning" as used in the present disclosure includes operations that provide, among other things, skin rejuvenation treatment, cellulite management treatment, body contouring procedures, acne treatment procedures, hair removal, and other skin or tissue treatments.

The term "current tissue toning protocol" means a protocol according to which the immediate tissue toning will be performed.

The term "terminations" and "terminations configured to couple energy to the tissue" as used in the present disclosure includes electrodes that couple RF energy to the tissue, transducers that couple ultrasound waves to the tissue, windows, lenses, and optical waveguides that facilitate skin by optical radiation exposure, and rollers or balls of the massaging device that couple kinetic energy to the skin, as well as similar devices that are able to communicate energy from a source to the tissue.

The term "computer" as used in the present disclosure means a device capable of receiving data or information, processing it, and delivering the data processing results to another device. As such, a computer may include, as non-limiting examples, a personal computer, a PDA computer, a mobile telephone, and similar devices. Typically, a computer as defined herein would have a display but, other forms of user feedback, prompting and user interface may also be used such a sound, voice detection, brail screens, or the like.

As used herein, the terms "person" and "subject" have the same meaning and refer to any human or animal subject, as well as synthetic objects.

As used herein, the terms "optical radiation sources" and "optical radiation emitters" have the same meaning and refer to any source or emitter of visible or non-visible optical radiation.

The terms "treatment" or "toning" as used in the present disclosure have the same meaning and are used interchangeable through the text of the disclosure.

The term "video" as used in the pressed disclosure is related to the visual presentation of information, usually on a display screen.

### BRIEF LIST OF DRAWINGS

For a better understanding of the system of the invention and the method disclosed herein, reference is made to the following description, taken in connection with the accompanying drawings, in which like reference characters refer to the same parts throughout the different views. The drawings are not necessarily to scale, emphasis instead being placed upon illustrating the principles of the method and/or apparatus.
Figure 1 is a schematic illustration of an exemplary embodiment of the present system for personal tissue toning.
Figure 2 is a schematic illustration of an exemplary computer display indicating locations of desired tissue affecting energy coupling.
Figure 3A is a schematic illustration of an exemplary embodiment of the present tissue toning device.
Figure 3B is a schematic illustration of the bottom view of an exemplary embodiment of the tissue toning device of Figure 3A.

### DETAILED DESCRIPTION OF EXEMPLARY EMBODIMENTS

In the following detailed description, reference is made to the accompanying drawings that form a part hereof and wherein like reference numerals denote like elements through the several views.

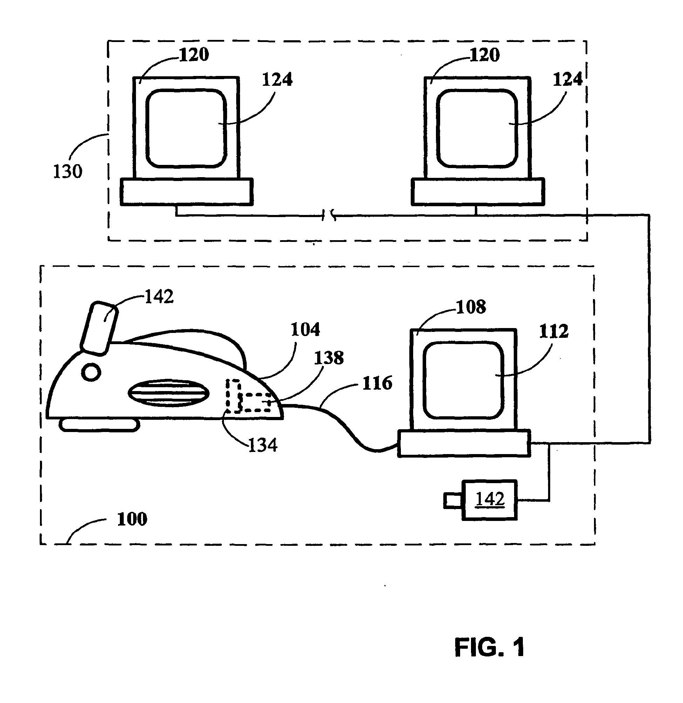

Reference is made to Figure 1 which is an exemplary embodiment of a personal tissue toning system. System 100 includes a tissue toning device 104, a computer 108 with a display 112, and a data transmission link 116 operative to transmit bidirectionally data between tissue toning device 104 and computer 108. The data transmission link 116 is shown as being a physical connection but, it will be appreciated that the connection may be wireless or optical as well. Computer 108, which will be termed the local computer may be of a variety of devices such as a personal computer, a palm computer, or a mobile telephone as non-limiting examples. Practically, any device having a processor and capable of processing data may be used in place of computer 108. Computer 108 is configured to communicate with a memory device or component 134 of tissue toning device 104 with the help of interface 138 and may be programmed to receive (or read) from memory 134 of device 104 the historical toning data accumulated in memory 134, analyze the historical toning data and issue a current tissue toning protocol. It should also be appreciated that the tissue toning device 104 may also include an embedded processing unit, for instance memory device 134 may be a microcontroller, and the processing unit may perform some or all of the analysis of the historical toning data and/or issue a current tissue toning protocol. Thus, the processing unit may be internal to the toning device 104, external in a computer or distributed between the two. The current tissue toning protocol optionally may include a video or virtual user image and suggestions for complementary skin care elements such as the use of various topical creams and lotions, vitamins or other tissue care related food additives or the like. Computer 108 downloads the current protocol to device 104 through the data transmission link 116. The communication between toning device 104 and computer 108 may be according to Ethernet, Bluetooth, or any other communication protocols and utilizing any of a variety of connection technologies. Local computer 108 would typically communicate with one or more remote computers 120 through dedicated communication links or via a network such as the Internet Remote computer 120, with its display 124, may be located at a remote site 130 to which a proper qualified medical personnel has access. The personnel may review the tissue toning protocols, their effects on the subject and issue additional recommendation.

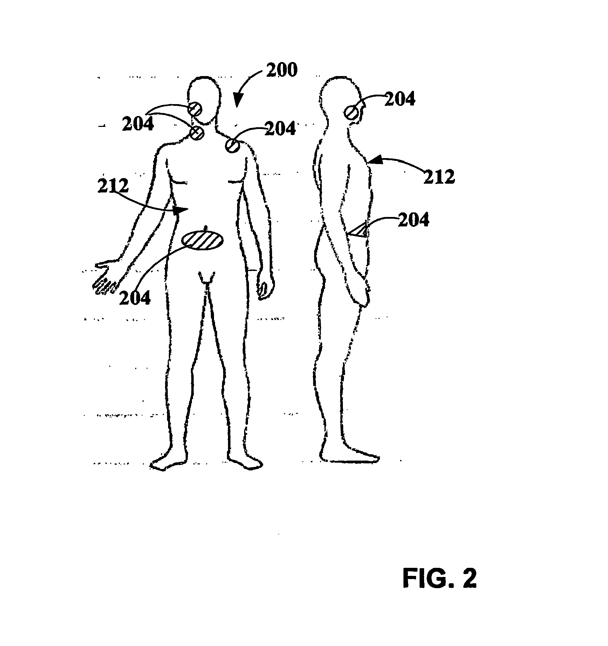

System 100 may include a digital or analog video camera 142 configured to take user 200 (Figure 2) images and/or video and display them on the local computer display 112, as well as to communicate the same to remote computer 120. Experienced medical personnel at the remote site 130 may mark on the video images of user 200 (Figure 2) target tissue segments locations 204 (Figure 2) to which according to the current protocol, tissue affecting energy should be applied or coupled. The user can optionally have the ability to modify or change the locations 204 to which the tissue affecting energy should be applied. In an alternative embodiment, the user image 200 may be a virtual symbolic image.

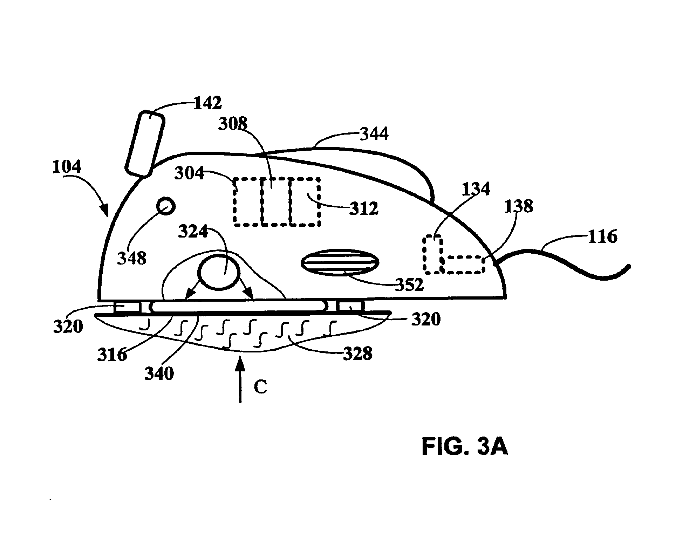

Tissue toning device 104 is configured to apply a tissue affecting energy to a target segment 204 of tissue 212 (Figure 2). Tissue toning device 104 typically includes at least a memory 134 (Figure 3A) configured to store and accumulate data related to historical or earlier performed tissue toning results and a computer interface 138 facilitating connection and communication with local computer 108, and an optional display 142. The toning history may include data indicating to the user the time of the earlier performed or historic tissue toning procedures, their duration, the tissue affecting energy parameters, segments of the body treated, and the expected toning results in textual or graphical representation. The user may display the segments of the body to be treated, and the expected toning results in textual or graphical representation on an optional display 142 of device 104. The display 142 may assist guiding the user in proper locations of tissue toning energy application.

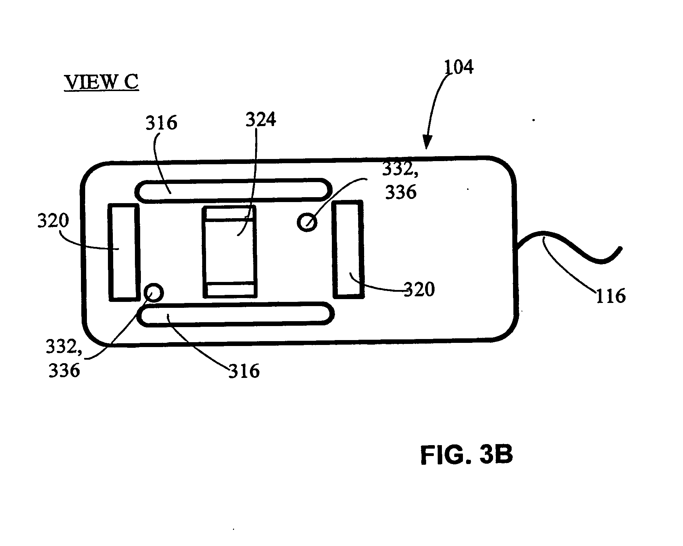

Figure 3 is a schematic illustration of an exemplary embodiment of the present tissue toning device. Tissue toning device 104 for personal skin toning further may include one or more types of terminations, which are elements configured to couple to the tissue, the tissue affecting energy provided by the respective energy sources. The energy sources may be incorporated in the tissue toning device or packed in a stand-alone housing (not shown). The sources of tissue affecting energy may be such as one or more optical radiation sources 304 shown by their power supplies, RF energy source 308, ultrasound waves source 312, and kinetic energy sources (not shown). Tissue toning device 104 may include any one of the tissue affecting energy sources 304, 308, or 312 and any combination of them. Terminations couple the tissue affecting energy to a segment 204 of user/subject 200 (Figure 2) tissue 212 to be toned. The terminations may be one or more RF electrodes 316, ultrasound transducers 320, and optical radiation sources 324, and optical radiation emitting and conveying elements, such as lamps, lenses, light guides, optical windows, and kinetic energy sources (not shown) such as massagers equipped with rollers or balls.

RF electrodes 316 may have elongated and curved bodies and may be solid, flexible, and hollow electrodes made of a heat conductive metal, or metal coated plastic, or composite material. RF electrodes 316 may be permanently attached to tissue toning device 104 or may be detachable from tissue toning device 104. Ultrasound transducers 320 may be conventional or phased array transducers. Optical radiation emitting elements 324 expose the target tissue segment to the radiation generated by the elements emitting optical radiation, such as incandescent lamps and lamps optimized for emission of red and infrared radiation and a reflector, Intense Pulse Light (IPL) source, a LED, and a laser diode (as non-limiting examples). Optionally, toning device 104 may include a massaging device (not shown) coupling to the target segment of the tissue with the help of rollers or balls kinetic energy. Tissue toning device 104 may include one or more of such sources or any combination of them.

Each of the terminations of tissue toning device 104 applies to the skin an appropriate type of skin affecting energy. One or more RF electrodes 316 that are in contact with skin 328 apply to the skin RF energy provided by RF energy sources. Ultrasound waves transducers 324 couple to the skin ultrasound energy generated by the ultrasound waves source 312, and the optical radiation emitting and conveying elements couple the optical radiation to skin 328 by exposing the skin to the energy generated by one or more optical radiation emitters 324.

Since all of the tissue affecting energies and methods disclosed alter the skin temperature at least to some degree, monitoring of the temperature is frequently used to control the toning process. Accordingly, tissue toning device 104 for personal skin toning may also include one or more tissue temperature sensors 332 (illustrated in Figure 3B) configured to measure the temperature of the target segment of the tissue. Even with performing the temperature monitoring, certain potential skin damage risk still exist because the sensor response time depends on heat conductivity from the skin to the sensor and inside the sensor, and may be too long and even damaging the skin before the sensor reduces or cuts off the skin heating power. In order to avoid such potential skin damage, temperature sensors 332 communicate the measured temperature to a processing circuit capable of deriving in the course of the tissue toning the rate of the temperature change of the target segment.

It has been experimentally discovered that the temperature change of the treated skin segment, and in particular a skin segment located between the RF electrodes and of the electrodes, being in contact with the skin depends on the applicator displacement speed. Heat transfer from the skin to the electrode and accordingly the temperature measured by the temperature sensor is largely dependent on the quality of the contact between the electrode and the skin. Differences in the quality of the contact could cause large variations in the temperature measurements. The quality of the skin-to-electrode contact may, for example, be monitored by monitoring the skin impedance and correcting the temperature change rate by an appropriate value or offset. Temperature sensor 332 may be of a variety of types, such as a thermistor, a thermocouple, or resistance temperature detectors as non-limiting examples. Further, temperature sensor 332 may be incorporated into a temperature probe 336 or into an electrode 316.

The displacement speed of the applicator can be determined in a variety of manners. On such non-limiting example includes the use of an accelerometer. Another non-limiting example includes the use of optical sensors that can determine movement relative to a target tissue segment

The coupling of tissue affecting energy to tissue 328 may be improved by application to the tissue of a gel 340 (shown in Figure 3A) that improves one or more of the tissue properties. For example, for RF coupling, the gel may have an electrical resistance higher than that of the tissue. Whereas for ultrasound waves, the coupling the gel may have acoustic coupling properties similar to those of the skin. The tissue toning device may optionally be equipped by a gel dispenser 344 that may be manually or automatically operated and configured to dispense over the skin/tissue 328 gel 340 that may have an electrical resistance higher than that of skin 328 and have acoustic coupling properties similar to those of the skin.

Although the user conducting treatment may be implementing the current protocol provided by the computer, the practical implementation of the protocol may differ from the recommended one and the user has to be given a continuous feedback on the status of interaction of the tissue affecting or heating energy with the tissue. The temperature change rate may be a basis for such feedback. For example, when tissue toning device 104 displacement speed is faster than the desired speed, the tissue does not receive enough heat and the treatment is not producing a desired effect and *vice versa.* The temperature change rate would be low for a rapidly displaced tissue toning device and *vice versa.* A visual or an audio signal indicator may be configured to provide the status of interaction of the tissue heating energy with the tissue. Accordingly, tissue toning device may include a visual signal indicator 348 that may be operative to indicate that the tissue toning device displacement speed is faster than the one set by the current protocol and/or that the tissue heating energy level is lower than the desired one. The audio indicator 352 may be operative to signify that the tissue toning device displacement speed is slower than the one set by the current protocol tissue toning device displacement speed and/or that the skin heating energy level is higher than the desired one (high temperature change rate) and may cause skin bums. Either the visual signal of different colors or the audio signal of different tones or a combination of them may be used in various embodiments.

The method of use of system 100 in tissue toning procedures is now described. For tissue treatment, tissue toning device 104 connects to a computer, which may be local computer 108 (Figure 1) or remote computer 120 and upon request, communicates or uploads to the computer the earlier performed or historic toning data presently stored in memory 134. The historical toning data may include sex and age of the treated subject, tissue affecting energy parameters of the earlier performed tissue toning, segments of the subject body that were treated, and what were the toning results in textual or graphical representation. The computer may also hold updated information on relevant topics from the medical field or the device manufacturer as well as studies on different populations and age groups.

The computer analyzes the toning data received and issues one or more current tissue toning protocols. The current tissue toning protocol includes at least one or more tissue affecting energies, their respective power, duration, and coupling location or target tissue segment 204 (Figure 2) on the subject 200 body. The segments of the subject body to be currently treated may be displayed in textual or graphical representation on the local computer display 112 or on the tissue toning device display 142. In case there is a need to consult with a medical specialist, communication with a company expert or an experienced user at the remote computer site 130 may be initiated, or a telephone call via conventional or Internet based lines may be placed to clear some of the issues that may exist.

The computer (local or remote) downloads the current tissue toning protocol into device 104 memory 134. Alternatively, local computer 108 (Figure 1) may control the tissue current tissue toning session. User 200 brings device 104 in contact with tissue 204 and couples to the skin/tissue the tissue affecting energy, which may be RF energy, optical radiation, and/or ultrasound waves or the like. Each of the energies may be coupled alone or, a combination of energies may be coupled simultaneously or in any desired order or combination to the tissue. By displacing or moving device 104 over the tissue, the user applies the current protocol to a target segment 204 and additional to be treated tissue segments of the subject 200 body. In order to facilitate the tissue toning procedure, the target segment 204 of the tissue may be displayed on the device 104 display 142 or on local computer 108 display 112. Concurrently with displaying the current toning location the expected current toning procedure results may be displayed on the same display.

The user/subject controls the tissue toning device displacement speed and the quality of at least one type of tissue affecting energy with the skin coupling by observing one of the signal indicators. The visual signal indicator 348 may be operative to indicate that the tissue toning device 104 displacement speed is faster than determined by the current protocol speed, and accordingly that the skin heating energy level is lower than necessary. The audio signal indicator 352 may be operative to signify that the tissue toning device displacement speed is slower than the displacement speed determined by the current protocol and accordingly that the skin heating energy level is higher than the desired one. For optimal toning results the subject adapts the speed so as to avoid any signal indicator activation.

## Claims

1. A personal tissue toning system (100) comprising:
a tissue toning device (104) for providing a personal skin toning procedure that applies tissue affecting energy to a target tissue segment, including a memory (134) for maintaining historical toning results and a computer interface (138);
at least one computer (108) with a user interface and a processing unit, wherein the computer (108) is communicatively coupled with the tissue toning device memory (134) to receive the historical toning data, and is configured to accumulate the historical toning data, to analyze the historical toning data and to issue a current tissue toning protocol;
a data transmission system (116) that is adapted to communicatively couple the tissue toning device (104) and the computer (108), whereby the computer is adapted to download the current tissue toning protocol to the tissue toning device (104) through the data transmission system (116);
at least one tissue temperature sensor (332) configured to measure the temperature of the target tissue segment during the toning procedure and communicate the measured temperature to the processing unit; and whereby the processing unit is operative to derive a rate of temperature change in the course of the toning procedure;
**characterized in that** the tissue toning device (104) also comprises means for measuring skin impedance and wherein said processing unit is also operative to derive from measured skin impedance the quality of skin-to-electrode contact and correct the temperature change rate by an appropriate value or offset in accordance to said derived quality of skin-to-electrode contact.

2. The personal tissue toning system according to claim 1, wherein the historical toning data includes the time that toning was performed, the duration of the toning, the tissue affecting energy parameters, the segment of the body treated, and the expected toning results.

3. The personal tissue toning system according to claim 1, also comprising a video camera (142) configured to take user images and/or video and at least one of display the images or video on a display (112) of the computer and communicate the images or video to a remote computer (120).

4. The personal tissue toning system according to claim 1, wherein the tissue toning device also comprises:
at least one termination (316, 320, 324) configured to couple to a target tissue segment, tissue affecting energy provided by at least one tissue affecting energy source (304, 308, 312);
at least one displacement speed sensor operative to determine displacement of said device relative to said target tissue segment and provide continuous feedback regarding the device displacement speed during the toning procedure to said processing unit; and
at least one indicator (348, 352) configured to indicate changes in the status of the interaction of said device and at least one type of tissue affecting energy with said target tissue segment based on said temperature and displacement speed feedbacks.

5. The tissue toning device (104) according to claim 4, wherein said at least one tissue affecting energy source is selected from a group of sources comprising an optical radiation source (304, 324), an RF energy source (308), kinetic energy source and an ultrasound waves source (312) and the terminations are selected from a group of terminations comprising RF electrodes (316), ultrasound transducers (320), optical radiation emitting and conveying elements, and a massaging device.

6. The tissue toning device (104) according to claim 5, wherein the RF electrode (316) is selected from a group of electrodes types comprising elongated bodies and curved bodies and wherein the electrodes further selected from a group of electrode types comprising solid, flexible, and hollow electrodes, with each electrode being made of a material selected from a group of materials comprising heal conductive metal, metal coated plastic and composite material and wherein the electrode is also detachable from the tissue toning device.

7. The tissue toning device according to claim 5, wherein the ultrasound transducer is a phased-array transducer.

8. The tissue toning device according to claim 5, wherein the optical radiation source (324) is selected from a group of sources comprising an incandescent lamp and a lamp optimized for emission of red and infrared radiation and a reflector, an Intense Pulse Light source, an LED and a laser diode.

9. The tissue toning device (104) according to claim 4, wherein the temperature sensor is selected from a group of sensors comprising thermistors, thermocouples, and resistance temperature detectors and wherein the temperature sensor is coupled to a temperature probe.

10. The tissue toning device (104) according to claim 4, wherein the indicators are at least one of a visual indicator (348) and an audio signal indicator (352) and wherein the visual signal indicator (348) is operative to indicate that the tissue toning device (104) displacement speed is faster than necessary and the audio signal indicator (352) is operative to signify that the tissue toning device (104) displacement speed is slower than the proper tissue toning device displacement speed.

11. The tissue toning device according to claim 10, wherein the visual signal indicator is operative to indicate that the skin heating energy level is lower than necessary, and the audio indicator is operative to signify that the skin heating energy level is higher than necessary.

12. The tissue toning device according to claim 1, wherein the computer (108) is also communicatively coupled with at least one of a remote computer (120) through at least one of a dedicated communication link and a network.

13. The tissue toning device according to claim 1, wherein the current tissue toning protocol comprises a video or virtual user image and suggestions for complementary skin care elements to be applied to the target tissue segment.

## Patentansprüche

1. Persönliches Gewebestraffungssystem (100), das Folgendes umfasst:
eine Gewebestraffungsvorrichtung (104) zur Bereitstellung eines persönlichen Hautstraffungsverfahrens, das gewebebeeinflussende Energie an ein Zielgewebesegment anlegt, wobei die Vorrichtung einen Speicher (134) zum Speichern historischer Straffungsergebnisse und eine Computerschnittstelle (138) umfasst;
mindestens einen Computer (108) mit einer Benutzerschnittstelle und einer Verarbeitungseinheit, wobei der Computer (108) kommunikativ mit dem Speicher (134) der Gewebestraffungsvorrichtung gekoppelt ist, um die historischen Straffungsdaten zu empfangen, und dafür konfiguriert ist, die historischen Straffungsdaten zu sammeln, die historischen Straffungsdaten zu analysieren und ein aktuelles Gewebestraffungsprotokoll auszugeben;
ein Datenübertragungssystem (116) das dafür ausgelegt ist, die Gewebestraffungsvorrichtung (104) und den Computer (108) kommunikativ miteinander zu koppeln, wobei der Computer dafür ausgelegt ist, das aktuelle Gewebestraffungsprotokoll über das Datenübertragungssystem (116) in die Gewebestraffungsvorrichtung (104) herunterzuladen;
mindestens einen Gewebetemperatursensor (332), der dafür konfiguriert ist, die Temperatur des Zielgewebesegments während des Straffungsverfahrens zu messen und die gemessene Temperatur zu der Verarbeitungseinheit zu übertragen; und wodurch die Verarbeitungseinheit dafür ausgelegt ist, eine Temperaturänderungsrate im Laufe des Straffungsverfahrens abzuleiten;
**dadurch gekennzeichnet, dass** die Gewebestraffungsvorrichtung (104) darüber hinaus Mittel zum Messen der Hautimpedanz umfasst und wobei die Verarbeitungseinheit darüber hinaus von der gemessenen Hautimpedanz die Qualität des Haut-Elektrode-Kontakts ableiten und die Temperaturänderungsrate um einen geeigneten Wert oder Versatz gemäß der abgeleiteten Qualität des Haut-Elektrode-Kontakts korrigieren kann.

2. Persönliches Gewebestraffungssystem nach Anspruch 1, wobei die historischen Straffungsdaten folgende Informationen umfassen: der Zeitpunkt, zu dem die Straffung durchgeführt wurde, die Dauer der Straffung, die gewebebeeinflussenden Energieparameter, das behandelte Körpersegment und die erwarteten Straffungsergebnisse.

3. Persönliches Gewebestraffungssystem nach Anspruch 1, das darüber hinaus eine Videokamera (142) umfasst, die dafür konfiguriert ist, Bilder und/oder Videos von dem Benutzer aufzuzeichnen und die Bilder oder Videos auf einer Anzeige (112) des Computers anzuzeigen und/oder die Bilder oder Videos zu einem entfernten Computer (120) zu übertragen.

4. Persönliches Gewebestraffungssystem nach Anspruch 1, wobei die Gewebestraffungsvorrichtung darüber hinaus Folgendes umfasst:
mindestens eine Abschlusseinheit (316, 320, 324), die dafür konfiguriert ist, mit einem Zielgewebesegment gekoppelt zu werden, wobei gewebebeeinflussende Energie durch mindestens eine Quelle (304, 308, 312) für gewebebeeinflussende Energie bereitgestellt wird;
mindestens einen Verschiebungsgeschwindigkeitssensor, der die Verschiebung der Vorrichtung im Verhältnis zu dem Zielgewebesegment bestimmen und der Verarbeitungseinheit eine kontinuierliche Rückmeldung über die Verschiebungsgeschwindigkeit der Vorrichtung während des Straffungsverfahrens bereitstellen kann; und
mindestens eine Anzeige (348, 352), die dafür konfiguriert ist, Änderungen des Status der Interaktion der Vorrichtung und mindestens einer Art von gewebebeeinflussender Energie mit dem Zielgewebesegment anzuzeigen und zwar basierend auf der Rückmeldung der Temperatur und der Verschiebungsgeschwindigkeit.

5. Gewebestraffungsvorrichtung (104) nach Anspruch 4, wobei die mindestens eine gewebebeeinflussende Energiequelle aus einer Gruppe von Quellen ausgewählt wird, die Folgendes umfasst: eine optische Strahlungsquelle (304, 324), eine HF-Energiequelle (308), eine kinetische Energiequelle und eine Ultraschallwellenquelle (312) und wobei die Abschlusseinheiten aus einer Gruppe von Abschlusseinheiten ausgewählt werden, die Folgendes umfassen: HF-Elektroden (316), Ultraschallwandler (320), optische strahlungsemittierende und -übertragende Elemente und eine Massagevorrichtung.

6. Gewebestraffungsvorrichtung (104) nach Anspruch 5, wobei die HF-Elektrode (316) aus einer Gruppe von Elektrodentypen ausgewählt wird, die längliche Körper und gekrümmte Körper umfasst, und wobei die Elektroden ferner aus einer Gruppe von Elektrodentypen ausgewählt werden, die feste, flexible und hohle Elektroden umfasst, wobei jede Elektrode aus einem Material besteht, das aus einer Gruppe von Materialien ausgewählt wird, die wärmeleitfähiges Metall, metallbeschichteten Kunststoff und Verbundmaterial umfasst, und wobei die Elektrode darüber hinaus von der Gewebestraffungsvorrichtung lösbar ist.

7. Gewebestraffungsvorrichtung nach Anspruch 5, wobei der Ultraschallwandler ein Phased-Array-Wandler ist.

8. Gewebestraffungsvorrichtung nach Anspruch 5, wobei die optische Strahlungsquelle (324) aus einer Gruppe von Quellen ausgewählt wird, die Folgendes umfasst: eine Glühlampe und eine Lampe, die für die Emission von roter und Infrarotstrahlung optimiert ist, und einen Reflektor, ein Intensivimpulslichtquelle, eine LED und eine Laserdiode.

9. Gewebestraffungsvorrichtung (104) nach Anspruch 4, wobei der Temperatursensor aus einer Gruppe von Sensoren ausgewählt wird, die Folgendes umfasst: Thermistoren, Thermoelemente und Widerstandstemperaturdetektoren und wobei der Temperatursensor mit einer Temperatursonde gekoppelt ist.

10. Gewebestraffungsvorrichtung (104) nach Anspruch 4, wobei die Anzeigen mindestens eine visuelle Anzeige (348) und/oder eine Audiosignalanzeige (352) sind und wobei die visuelle Signalanzeige (348) anzeigen kann, dass die Verschiebegeschwindigkeit der Gewebestraffungsvorrichtung (104) schneller als notwendig ist und wobei die Audiosignalanzeige (352) anzeigen kann, dass die Verschiebegeschwindigkeit der Gewebestraffungsvorrichtung (104) langsamer ist als die korrekte Verschiebegeschwindigkeit der Gewebestraffungsvorrichtung.

11. Gewebestraffungsvorrichtung nach Anspruch 10, wobei die visuelle Signalanzeige anzeigen kann, dass der Hauterwärmungspegel niedriger als notwendig ist und die Audioanzeige anzeigen kann, dass der Hauterwärmungspegel höher als notwendig ist.

12. Gewebestraffungsvorrichtung nach Anspruch 1, wobei der Computer (108) darüber hinaus mit mindestens einem entfernten Computer (120) durch mindestens eine dedizierte Kommunikationsverbindung und/oder ein Netzwerk kommunikativ gekoppelt ist.

13. Gewebestraffungsvorrichtung nach Anspruch 1, wobei das Stromgewebestraffungsprotokoll ein Video oder ein virtuelles Benutzerbild sowie Vorschläge für ergänzende Hautpflegeelemente, die auf das Zielgewebesegment angewendet werden können, umfasst.

## Revendications

1. Un système de coloration de tissu personnel (100) comprenant :
un dispositif de coloration de tissu (104) destiné à la fourniture d'une procédure de coloration de la peau personnelle qui applique une énergie affectant un tissu à un segment de tissu cible, comprenant une mémoire (134) destinée à l'entretien d'un historique de résultats de coloration et une interface informatique (138),
au moins un ordinateur (108) avec une interface utilisateur et une unité de traitement, où l'ordinateur (108) est couplé en communication avec la mémoire du dispositif de coloration de tissu (134) de façon à recevoir l'historique de données de coloration et est configuré de façon à accumuler l'historique de données de coloration de façon à analyser l'historique de données de coloration et produire un protocole de coloration de tissu actuel,
un système de transmission de données (116) qui est adapté de façon à coupler en communication le dispositif de coloration de tissu (104) et l'ordinateur (108), grâce à quoi l'ordinateur est adapté de façon à télécharger le protocole de coloration de tissu actuel vers le dispositif de coloration de tissu (104) par l'intermédiaire du système de transmission de données (116),
au moins un capteur de température de tissu (332) configuré de façon à mesurer la température du segment de tissu cible au cours de la procédure de coloration et à communiquer la température mesurée à l'unité de traitement, et grâce à quoi l'unité de traitement est conçue de façon à dériver une vitesse de changement de température au cours de la procédure de coloration,
**caractérisé en ce que** le dispositif de coloration de tissu (104) comprend également un moyen destiné à la mesure d'une impédance de peau et où ladite unité de traitement est également conçue de façon à dériver à partir d'une impédance de peau mesurée la qualité d'un contact peau-électrode et à corriger la vitesse de changement de température par une valeur appropriée ou décalée en fonction de ladite qualité dérivée d'un contact peau-électrode.

2. Le système de coloration de tissu personnel selon la Revendication 1, où l'historique de données de coloration comprend l'heure à laquelle la coloration a été exécutée, la durée de la coloration, les paramètres d'énergie affectant un tissu, le segment du corps traité et les résultats de coloration attendus.

3. Le système de coloration de tissu personnel selon la Revendication 1, comprenant également une caméra vidéo (142) configurée de façon à prendre des images et/ou une vidéo d'utilisateur et au moins une opération parmi l'affichage des images ou de la vidéo sur un dispositif d'affichage (112) de l'ordinateur et la communication des images ou de la vidéo à un ordinateur distant (120).

4. Le système de coloration de tissu personnel selon la Revendication 1, où le dispositif de coloration de tissu comprend également :
au moins une borne (316, 320, 324) configurée de façon à se coupler à un segment de tissu cible, une énergie affectant un tissu étant fournie par au moins une source d'énergie affectant un tissu (304, 308, 312),
au moins un capteur de vitesse de déplacement conçu de façon à déterminer un déplacement dudit dispositif par rapport audit segment de tissu cible et à fournir des informations en retour en continu concernant la vitesse de déplacement du dispositif au cours de la procédure de coloration à ladite unité de traitement, et
au moins un indicateur (348, 352) configuré de façon à indiquer des modifications dans l'état de l'interaction dudit dispositif et au moins un type d'énergie affectant un tissu avec ledit segment de tissu cible en fonction desdites informations en retour de température et de vitesse de déplacement.

5. Le dispositif de coloration de tissu (104) selon la Revendication 4, où ladite au moins une source d'énergie affectant un tissu est sélectionnée dans un groupe de sources comprenant une source de rayonnement optique (304, 324), une source d'énergie RF (308), une source d'énergie cinétique et une source d'ondes ultrasonores (312), et les bornes sont sélectionnées dans un groupe de bornes comprenant des électrodes RF (316), des transducteurs ultrasonores (320), des éléments d'émission et d'acheminement de rayonnement optique et un dispositif de massage.

6. Le dispositif de coloration de tissu (104) selon la Revendication 5, où l'électrode RF (316) est sélectionnée dans un groupe de types d'électrodes comprenant des corps allongés et des corps incurvés et où les électrodes sont en outre sélectionnées dans un groupe de types d'électrode comprenant des électrodes solides, flexibles et creuses, chaque électrode étant composée d'un matériau sélectionné dans un groupe de matériaux comprenant un métal thermoconducteur, un plastique revêtu de métal et un matériau composite et où l'électrode est également détachable du dispositif de coloration de tissu.

7. Le dispositif de coloration de tissu selon la Revendication 5, où le transducteur ultrasonore est un transducteur à commande de phase.

8. Le dispositif de coloration de tissu selon la Revendication 5, où la source de rayonnement optique (324) est sélectionnée dans un groupe de sources comprenant une lampe à incandescence et une lampe optimisée pour l'émission d'un rayonnement rouge et infrarouge et un réflecteur, une source de lumière pulsée intense, une LED et une diode laser.

9. Le dispositif de coloration de tissu (104) selon la Revendication 4, où le capteur de température est sélectionné dans un groupe de capteurs comprenant des thermistors, des thermocouples et des détecteurs de température à résistance et où le capteur de température est couplé à une sonde de température.

10. Le dispositif de coloration de tissu (104) selon la Revendication 4, où les indicateurs sont au moins un indicateur parmi un indicateur de signal visuel (348) et un indicateur de signal audio (352) et où l'indicateur de signal visuel (348) est conçu de façon à indiquer que la vitesse de déplacement du dispositif de coloration de tissu (104) est plus rapide que nécessaire et l'indicateur de signal audio (352) est conçu de façon à signaler que la vitesse de déplacement du dispositif de coloration de tissu (104) est inférieure à la vitesse de déplacement propre du dispositif de coloration de tissu.

11. Le dispositif de coloration de tissu selon la Revendication 10, où l'indicateur de signal visuel est conçu de façon à indiquer que le niveau d'énergie de chauffage de la peau est plus bas que nécessaire, et l'indicateur audio est conçu de façon à signaler que le niveau d'énergie de chauffage de la peau est plus élevé que nécessaire.

12. Le dispositif de coloration de tissu selon la Revendication 1, où l'ordinateur (108) est également couplé en communication avec au moins un élément parmi un ordinateur distant (120) par l'intermédiaire d'au moins un élément parmi une liaison de communication dédiée et un réseau.

13. Le dispositif de coloration de tissu selon la Revendication 1, où le protocole de coloration de tissu actuel comprend une image ou une vidéo d'utilisateur virtuel et des suggestions d'éléments de soin de la peau complémentaires à appliquer au segment de tissu cible.
